Europäisches Patentamt

European Patent Office

Office européen des brevets

(11) Publication number: **0 051 954**
**B1**

(12) **EUROPEAN PATENT SPECIFICATION**

(45) Date of publication of patent specification: **02.05.85**

(21) Application number: **81305048.1**

(22) Date of filing: **26.10.81**

(51) Int. Cl.⁴: **A 61 F 2/08**

(54) Structure for in vivo implantation.

(30) Priority: **06.11.80 US 204529**

(43) Date of publication of application:
**19.05.82 Bulletin 82/20**

(45) Publication of the grant of the patent:
**02.05.85 Bulletin 85/18**

(84) Designated Contracting States:
**CH DE FR GB LI**

(56) References cited:
**FR-A-2 135 825**
**FR-A-2 395 012**
**US-A-3 973 277**
**US-A-4 127 902**
**US-A-4 129 470**

(73) Proprietor: **Homsy, Charles A.**
**11526 Raintree Cir.**
**Houston Texas 77024 (US)**

(72) Inventor: **Homsy, Charles A.**
**11526 Raintree Cir.**
**Houston Texas 77024 (US)**

(74) Representative: **Lambert, Hugh Richmond et al**
**D. YOUNG & CO. 10 Staple Inn**
**London, WC1V 7RD (GB)**

Courier Press, Leamington Spa, England.

## Description

The present invention relates to an improved tension member suitable for in vivo implantation e.g. as a ligament or a tendon.

Some ligament and tendon damage is not repairable by surgery except by transplant or by the implantation of a replacement structure i.e. an artificial ligament or tendon. One requirement for such replacement structures is that they should have extended fatigue resistance so that their replacement by surgery is not required during the normal patient life.

The problems of attachment of the ends of the implanted tension member, of causing a tunnel to form to allow sliding movement of the implanted tension member and of biocompatibility have been solved by the structure disclosed in U.S. Patent No. 4,127,902. However, increased fatigue resistance is advantageous in such implantable structures.

The present invention seeks to provide an improved implantable tension member which has improved flexural characteristics and fatigue resistance and which provides a potential for longer use when impanted in vivo as a substitute ligament or tendon.

According to the present invention, a structure suitable for in vivo implantation as a tension member is provided comprising a plurality of parallel strands of a biocompatible polymer bonded together at their opposite ends by a film of biocompatible bonding resin and surrounded intermediate their bonded ends by a sleeve of biocompatible polymeric material within which said strands are slidable both with respect to each other and to the sleeve, and which sleeve is bonded at at least one end to the film of resin binding the strands together.

Such a structure allows each of the strands to carry a substantially equal share of the load independent of the flexing of the structure.

The invention is further explained with reference to the accompanying drawings, wherein:—

Figure 1 is a plan view of a tension member according to the present invention at an intermediate stage of manufacture, i.e. before removal of the transverse strands of the fabric and folding over of the sealed ends to form an artificial ligament or tendon in accordance with the invention;

Figure 2 is a plan view, partially cut away, of a preferred form of a tension member in accordance with the invention with portions broken away to show the interior structure;

Figure 3 is a sectional view of the tension member taken along line 3—3 in Figure 2;

Figure 4 is a view of the initial form of the structure of Figure 5, illustrating the longitudinal fibers in a loop;

Figure 5 is a view of another form of tension member in accordance with the invention and useful as an artificial tendon.

Figure 6 is a partial view of the structure of Figure 4 showing a means for attaching the artificial tendon or tension member in its implanted position.

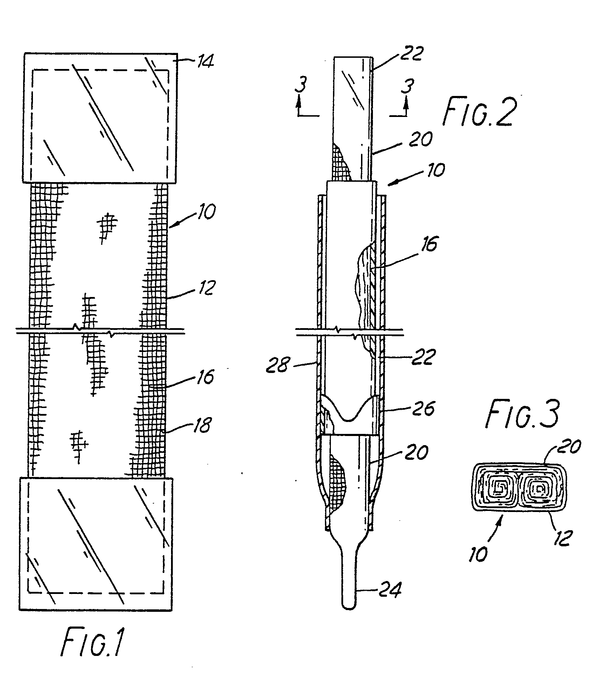

As shown in Figure 1, the implantable tension member 10 of the present invention is formed from a strip 12 the opposite ends of which are bonded by films 14. Fabric 12 and films 14 are both of biocompatible material and fabric 12 is heat stable at the temperature used for the bonding of films 14 on the ends of fabric. The material of the films 14 is preferred to be a perfluorocarbon, a perfluoroalkoxy fluorocarbon, a high molecular weight polyethylene, a hydrohalocarbon or a halocarbon.

The preferred material of the fabric 12 is 'Leno-weave' fabric of polyamide (nylon) material, such as polyaramide, sold by DuPont Company under the trade mark 'Nomex'. Such fabric is available from Stern & Stern, Inc. of Hornell, New York as fabric type HT-63-30. Other fabrics suitable for the invention are polytetrafluoroethylene fabric, polyimide fabric and polyester fabric. In such fabrics, the strands or fibers extending in one direction (longitudinal or warp strands 16) are disposed in twisted pairs and the fibers or strands extending in the perpendicular or transverse direction (fill strands 18) pass through the twisted pairs of the warp strands 16.

As used herein, the terms strand and fibre are used to mean the elements of the fabric and are not intended to exclude monofilaments.

Before or after the films 14 are bonded to the ends of the fabric 12, the fill (weft) strands 18 are removed from the intermediate portion of the fabric strip 12 which is between the films 14 so that only warp strands 16 remain between the bonded ends after bonding. This leaves a structure of a plurality of parallel strands with their ends bonded by the films 14.

Since it is desired that strands 16 be easily slidable with respect to each other, they are preferably coated with a biocompatible resin to improve their lubricity. This is accomplished by immersing strands 16 in a slurry of a suitable polytetrafluoroethylene resin such as is available from DuPont Company under the trade mark Teflon T-6 in a suitable wetting liquid, such as isoparaffine solvent marketed by Exxon Chemical Company under the mark Isopar E. The slurry is prepared by adding 9.9 grams of the Teflon T-6 resin to 600 cc of Isopar E and blending for sufficient time (5 minutes) to develop an intimate dispersion. With the blender on, the stands are dipped into the slurry for a period of time sufficient to coat strands 16, as for example one-half minute to five minutes. Strands 16 are removed from the slurry and the solvent evaporated, for example, by drying in an oven for two hours. This procedure has been found to provide a substantially uniform coating of the strands with particles of the Teflon T-6 resin.

Thereafter, the film coated ends and strands 16 are folded into a compact rectangular shape as shown in Figure 3 and inserted into a protective sleeve 22 of a suitable biocompatible polymer, said sleeve being bonded at at least one end to one of the films 14 of bonding resin. The sleeve 22 is thus secured to the structure whilst permitting relative sliding movement and flexing of the individual strands 16 within the protective sleeve. The protective sleeve 22 is preferably a porous biocompatible polymer e.g. of Teflon (Registered Trade Mark) TFE, wherein the porosity of the sleeve is in the range from 50 to 90 percent by volume and preferably 60 percent. The material of such sleeve is preferred to be the material described in my European Application No. 81305049 filed concurrently herewith (Publication No. EP—A—0051955), and to which reference should be made for full details. Briefly, however, such material is a porous, fibrous structure consisting of polytetrafluoroethylene fibers in a matrix of polytetrafluoroethylene resin obtained by forming a blend of the polytetrafluoroethylene fibers and the polytetrafluoroethylene and from 50—90% by volume of a particulate pore-forming agent, e.g. sodium chloride, compacting and sintering the blend to form a fused sheet structure and leaching out the pore-forming agent.

Sleeve 22 is formed around a mandrel by heat and pressure during the sintering operation. During this process the outer surface of sleeve 22 is annealed so that some of the resin melts and covers the outer surface to reduce its pore openings and thereby provide a surface which inhibits the ingrowth of tissue. However, when maximum surface porosity is desired to enhance tissue ingrowth, as for example when the structure is to be used for replacement of the collateral ligaments of the knee, the occluding resin skin on the sleeve can be removed by mild mechanical abrasion. Conversely the surface porosity can be totally obliterated in order for the structure to be suitable as a substitute tendon. One method of achieving this is by coating the surface of the sleeve with medical grade silicone rubber for example by dipping or spray coating the sleeve with a dispersion of medical grade silicone rubber in a nonpolar vehicle such as a halogenated ethylene, e.g. chlorthene solvent. It has been unexpectedly found that such thin coatings of silicone rubber are tenaciously bound to the surface of the sleeve. Since polytetrafluoroethylene is well known for its resistance to adhesion to other materials especially non-polar materials such as silicone rubber, the tenacity of attachment of the coating on the sleeve is surprising.

Furthermore, it has been found that vacuum impregnation of the entire porosity of the sleeve with said dispersion of silicone rubber followed by appropriate drying and curing of the rubber yields a novel composite of PTFE polymer and silicone rubber elastomer. This composite retains the flexibility of the porous PTFE sleeve material but exhibits enhanced resiliency, toughness and flexural fatigue behaviour. By appropriate adjustment of the vacuum impregnation conditions the depth of impregnation of the silicone rubber into the porous PTFE sleeve material can be controlled and can produce a silicone coating more tenaciously bound than that achieved by the simple dipping or spraying procedures described above.

Sleeve 22 is positioned around the formed structure and the short length of each end, not less than 1 cm is bonded by heat to the film covered portion of the structure. Surface occlusion with silicone rubber may be carried out after this step.

Alternatively, the sleeve 22 may be wholly of silicone rubber, or other biocompatible polymer.

Thus, a complete structure is formed suitable for implantation in vivo in substitute for a ligament. The strands 16 within sleeve 22 are free to move therein to adjust their positions so that with the flexural loading of the structure along this portion of the structure, stress is substantially uniformly distributed to the strands in all positions of flexion. Also, sleeve 22 protects strands 16 from abrasion or other damage by reason of engagement by or between tissue elements, such as bones, cartilage or soft tissue. One end of substitute ligament structure is formed in a reduced size to provide a leader 24 which eases the insertion of the end through a hole in a bone.

As shown in Figure 2, a means is provided on the ligament prosthesis to protect sleeve 22 from damage in the area where the structure enters or leaves a hole in bone. Such protection means is advisable since both ends of drilled holes or tunnels usually are not always available to the surgeon for rounding the entrances and exits to and from such tunnels. The preferred form of such protection means is skirt 26 of previously described polytetrafluoroethylene in the form of a tube four to five centimeters long with one to two centimeters at either end of skirt 26 being bonded to the exterior of sleeve 22 at either or both ends of sleeve 22 depending upon the mode of implantation. Skirt 26 may be shaped so that it does not totally encircle sleeve 22 along the free (unbonded) length as shown in Figure 2 or may be a tube of uniform length.

An additional protection means is provided in the form of protective sheath 28 which is tubular in shape and is formed of Nomex fabric and a perfluorocarbon film such as that sold by DuPont under the trade mark Telflon FEP with the FEP film overlapping but with a longitudinal gap between the Nomex fabric. Sheath 28 is secured around the portion of structure close to leader 24 and extends over a substantial portion of sleeve 22 so that it is protected in threading into its implanted position. Then leader 24 is cut from the positioned structure and the bonded portion of sheath 28 is removed with such cut and sheath 28 is easily cut longitudinally in that film portion which is free of fabric and removed from the implant structure and discarded.

In order to provide in situ bonding of the ends of the tension member in vivo the opposite ends of the structure are preferably further coated with a layer 20 of porous biocompatible polymeric material which promotes the ingrowth of tissue within the pores of the material. Such a material, and a suitable anchoring arrangement for the tension members of the present invention are described in my U.S. Patents 3,992,725 and 4,129,470, to which reference should be made. Broadly speaking that material is a fibrous polymeric material having a critical surface tension in the range 25—80 dynes per centimeter and preferably comprising a blend of polytetrafluoroethylene and carbon fibers in a porous polytetra fluoroethylene resin matrix obtained by compacting and sintering a blend of polytetrafluoroethylene fibers, carbon fibers, particulate polytetrafluoroethylene resin and a particulate pore-forming agent, and subsequently leaching out the pore forming agent from the sintered matrix. Another suitable ingrowth promoting material is a velour material of polyester fiber.

Alternative means of anchoring or joining the tension structure of this invention to *in situ* tissue may also be employed.

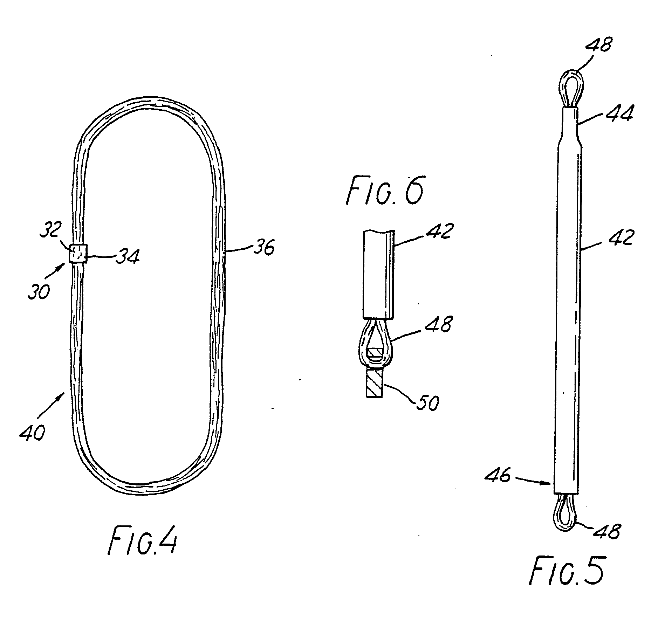

A similar structure 30 suitable for *in vivo* implantation as a substitute tendon is shown in Figures 4, 5 and 6. Fabric 32 is similar to fabric 12. Teflon FEP film 34 is bonded to the ends of fabric 32 and then the two ends are bonded together to form the loop 40. The transverse strands of fabric 32 are removed either after film 34 is bonded to its ends or after loop 40 is formed to leave the longitudinal strands 36. Strands 36 are immersed in the Teflon T-6-Isopar-E slurry for coating as previously described. Loop 40 is inserted into tube 42 with fabric ends 32 and film 34 immediately within one end of tube 42. Such tubes are easily flexible and may be formed of non-porous material such as silicone rubber or porous material having no external porosity or only small dimension exterior porosity not efficient for tissue ingrowth. Tube 42 is approximately three millimeters in diameter and of sufficient length for the particular tendon application. One end 44 of tube 42 is bonded to film 34 and fabric ends 32 by suitable heat and pressure or silicone adhesive. The other end 46 is sealed with a silicone rubber. Thus, entry of materials or tissue into the interior of the tube 42 is prevented.

Tube 42 may be made from the same material as the material of sleeve 22 previously described. However, for implantation of such material in a substitute tendon application, any pores in the outer surface of tube 42 are occluded by annealing in the sintering process or by coating with silicone rubber as previously described. This provides a surface which inhibits the ingrowth of tissue so that the substitute tendon is not prevented from free sliding movement in its implanted position. Tube 42 may also be a polytetrafluoroethylene vascular graft material sold by W. L. Gore and Associates of Flagstaff, Arizona under the mark Gore-Tex. This material must also have external pores occluded by coating with silicone rubber as previously described or other suitable means.

Structure 30, as shown in Figure 5, includes strands 36 extending from the ends of tube 42 in the form of loops 48. Ingrowth material 50 may be secured in one or both of loops 48 by silicone rubber as shown in Figure 6. Ingrowth material 50 is preferred to be the porous material of graphite fibers bonded with tetrafluoroethylene as disclosed in my prior U.S. Patent No. 3,992,725. A material of polyester fiber velour would also be suitable.

Thus, tendon replacement structure 30 has a plurality of strands 36 having sufficient lubricity to adjust their positions relative to each other and are contained within tube 42 which is sufficiently large and flexible to allow such position adjustment so that each strand carries substantially all of its share of the load in all positions of the implanted structure, reduces interfiber wear and the strands are protected from damage by rubbing against bone or being caught between bones and crushed.

It is preferred that the tube or sleeve in which the strands are positioned have an internal cross-sectional area which is approximately twice the cross-sectional area occupied by the strands so that the strands are not restricted from their desired position adjustment to assume their share of the load.

It is understood that all materials used in the improved tension members of this invention are to be biocompatible.

**Claims**

1. A tension member for in vivo implantation characterised in that it comprises a plurality of parallel strands (16, 36) of a biocompatible polymer bonded together at their opposite ends by a bonding film (14, 34) of biocompatible bonding resin in which the ends of the strands are embedded, and a protective sleeve (22, 42) of biocompatible polymeric material surrounding said strands intermediate their bonded ends and bonded at at least one end to a said film of bonding resin, the said strands (16, 36) intermediate their bonded ends being readily slidable with respect to each other and with respect to the surrounding sleeve.

2. A tension member according to claim 1, characterised in that the said plurality of parallel strands (16, 36) are provided by the warp strands of a length of woven fabric of biocompatible fibres, the ends of which fabric are bonded by said films (14, 34) of bonding resin, and the weft strands of which have been removed from the length of fabric intermediate said bonded ends, thereby leave said warp strands in separate parallel array.

3. A tension member according to claim 1 or 2, characterised in that said parallel strands (16, 36) are lubricated by coating with a surface coating of biocompatible polytetrafluoroethylene resin.

4. A tension member according to claim 1, 2 or 3, characterised in that said parallel strands (36) are formed into an endless loop (40) and joined at their opposite ends by a common film (34) of biocompatible bonding resin in which the ends of said strands are embedded, said loop being inserted in said sleeve (42) with its opposite ends projecting therefrom to form anchoring loops for attachment of the tension member when making the implant.

5. A tension member according to claim 4, wherein one or both exposed ends of said loop is or are engaged with an anchoring member (50) of porous biocompatible polymeric material which promotes the ingrowth of tissue in the material when placed *in situ*.

6. A tension member according to any one of claims 1—5, characterised in that the protective sleeve (22) is of a porous biocompatible polymer.

7. A tension member according to claim 6, characterised in that the external surface of the protective sleeve (22) is treated to resist ingrowth of tissue into the sleeve.

8. A tension member according to claim 7, wherein the external surface of the sleeve (22) is annealed.

9. A tension member according to claim 7, wherein at least the surface pores of the sleeve (22) are occluded with silicone rubber.

10. A tension member according to claim 8, wherein the porous sleeve is substantially completely impregnated with silicone rubber.

11. A tension member according to claim 6, wherein the protective sleeve (22) has an open porous surface which promotes the ingrowth of tissue into the sleeve.

12. A tension member according to any one of claims 6—11, wherein the protective sleeve (22) is formed of a biocompatible polymeric material comprising fibers of polytetrafluoroethylene in a porous matrix of polytetrafluoroethylene bonding resin.

13. A tension member according to any one of claims 1—5, wherein said sleeve is of silicone rubber.

14. A tension member according to any one of the preceding claims, wherein the parallel strands (16, 36) are of polyamide, polyimide, polytetrafluoroethylene or polyester and said bonding film (14, 34) is a perfluorocarbon polymer, a perfluoroalkoxy fluorocarbon polymer or high molecular weight polyethylene.

15. A tension member according to any one of claims 1—3 or 6—14 as dependent thereon, wherein the ends of the structure adjacent the bonding films (14) are coated with a layer (20) of porous biocompatible polymeric material which promotes the ingrowth of tissue within the pores of the polymeric material.

16. A tension member according to claim 5 or 15, wherein the porous biocompatible polymeric material comprises a blend of polytetrafluoroethylene and carbon fibers in a porous matrix of polytetrafluoroethylene resin.

17. A tension member according to claim 5 or 15, wherein the porous polymeric material is a polyester fiber velour.

18. A tension member according to any one of the preceding claims, characterised in that at least one end of the sleeve 22 is protected by a protective skirt (26) of biocompatible material mounted around said sleeve and extending along at least part of the length thereof.

19. A tension member according to claim 18, characterised in that said skirt (26) comprises a tubular member coaxial with the end of said sleeve (22) and bonded thereto.

20. A tension member according to any one of the preceding claims, characterised in that at least one end of the structure is externally protected by a removable sheath (28) to protect said sleeve from damage prior to or during implantation and removable from said structure after implantation.

21. A tension member according to claim 20, characterised in that the externally protected end of the structure is of a reduced dimension to provide a leader (24) for insertion of the structure during implantation, said sheath (28) being bonded to said leader and removable therewith after implantation of the structure.

**Revendications**

1. Un élément de tension pour l'implantation in vivo, caractérisé en ce qu'il comprend plusieurs brins parallèles (16, 36) en polymère biocompatible unis ensemble à leurs extrémités opposées par une pellicule d'union (14, 34) en résine d'union biocompatible dans laquelle les extrémités des brins sont incorporées, et une gaine protectrice (22, 42) d'une matière polymère biocompatible entourant lesdits brins entre leurs extrémités unies et unie à au moins une extrémité à une dite pellicule de résine d'union, lesdits brins (16, 36) entre leurs extrémités unies pouvant glisser facilement les uns par rapport aux autres et par rapport à la gaine qui les entoure.

2. Un élément de tension selon la revendication 1, caractérisé en ce que lesdits plusieurs brins parallèles (16, 36) sont constituées par les brins de chaîne d'une longueur de tissu tissé en fibres biocompatibles, les extrémités du tissu étant unies par lesdites pellicules (14, 34) de résine d'union, et les brins de trame ont été éliminés de la longueur du tissu intermédiaire auxdites extrémités unies, pour laisser lesdits brins de chaîne sous forme d'un alignement parallèle séparé.

3. Un élément de tension selon la revendication 1 ou 2, caractérisé en ce que lesdits brins parallèles (16, 36) sont lubrifiés par revêtement avec un revêtement superficiel d'une résine de polytétrafluoroéthylène biocompatible.

4. Un élément de tension selon la revendication 1, 2 ou 3, caractérisé en ce que lesdits brins parallèles (36) sont formés en une boucle sans fin (40) et unis à leurs extrémités opposées par une pellicule commune (34) en résine d'union biocompatible dans laquelle les extrémités desdits brins sont incorporées, ladite boucle étant insérée dans ladite gaine (42) avec ses extrémités opposées en dépassant pour former des boucles d'ancrage pour la fixation de l'élément de tension lors de la réalisation de l'implant.

5. Un élément de tension selon la revendication 4, dans lequel l'une ou les deux extrémités apparentes de ladite boucle est ou sont engagées avec un élément d'ancrage (50) en matière polymère biocompatible poreuse qui favorise la croissance intérieure d'un tissu dans la matière lorsqu'elle est placée in situ.

6. Un élément de tension selon l'une quelconque des revendications 1 à 5, caractérisé en ce que la gaine protectrice (22) est en un polymère biocompatible poreux.

7. Un élément de tension selon la revendication 6, caractérisé en ce que la surface externe de la gaine protectrice (22) est traitée pour résister à la croissance d'un tissu dans la gaine.

8. Un élément de tension selon la revendication 7, dans lequel la surface externe de la gaine (22) est recuite.

9. Un élément de tension selon la revendication 7, dans lequel au moins les pores superficiels de la gaine (22) sont obturés par un caoutchouc de silicone.

10. Un élément de tension selon la revendication 8, dans lequel la gaine poreuse est essentiellement totalement imprégnée d'un caoutchouc de silicone.

11. Un élément de tension selon la revendication 6, dans lequel la gaine protectrice (22) a une surface poreuse ouverte qui favorise la croissance d'un tissu dans la gaine.

12. Un élément de tension selon l'une quelconque des revendications 6 à 11, dans lequel la gaine protectrice (22) est formée d'une matière polymère biocompatible comprenant des fibres de polytétrafluoroéthylène dans une matrice poreuse de résine d'union de polytétrafluoroéthylène.

13. Un élément de tension selon l'une quelconque des revendications 1 à 5, dans lequel ladite gaine est en caoutchouc de silicone.

14. Un élément de tension selon l'une quelconque des revendications précédentes, dans lequel les brins parallèles (16, 36) sont en polyamide, polyimide, polytétrafluoroéthylène ou polyester, et ladite pellicule d'union (14, 34) est un polymère de perfluorocarbure, un polymère de perfluoroalcoxyfluorocarbure ou un polyéthylène de poids moléculaire élevé.

15. Un élément de tension selon l'une quelconque des revendications 1 à 3 ou 6 à 14 lorsqu'elles leur sont subordonnées, dans lequel les extrémités de la structure adjacente aux pellicules d'union (14) sont revêtues d'une couche (20) d'une matière polymère biocompatible poreuse qui favorise la croissance du tissu dans les pores de la matière polymère.

16. Un élément de tension selon la revendication 5 ou 15, dans lequel la matière polymère biocompatible poreuse est constituée d'un mélange de fibres de polytétrafluoroéthylène et de carbone dans une matrice poreuse de résine de polytétrafluoroéthylène.

17. Un élément de tension selon la revendication 5 ou 15, dans lequel la matière polymère poreuse est un velours de fibres de polyester.

18. Un élément de tension selon l'une quelconque des revendications précédentes, caractérisé en ce qu'au moins une extrémité de la gaine (22) est protégée par un manchon protecteur (26) en matière biocompatible monté autour de ladite gaine et s'étendant le long d'au moins une partie de la longueur de celle-ci.

19. Un élément de tension selon la revendication 18, caractérisé en ce que ledit manchon (26) comprend un élément tubulaire coxial avec l'extrémité de ladite gaine (22) et uni à elle.

20. Un élément de tension selon l'une quelconque des revendications précédentes, caractérisé en ce qu'au moins une extrémité de la structure est protégée extérieurement par une enveloppe amovible (28) pour protéger ladite gaine contre les altérations avant ou pendant l'implantation et pouvant être éliminée de ladite structure après l'implantation.

21. Un élément de tension selon la revendication 20, caractérisé en ce que l'extrémité protégée extérieurement de la structure a une dimension réduite pour former un guide (24) pour l'insertion de la structure au cours de l'implantation, ladite enveloppe (28) étant unie audit guide et pouvant en être séparée après l'implantation de la structure.

**entansprüche**

1. Zugglied für eine Implantation in vivo, dadurch gekennzeichnet, daß es mahrere parallele Stränge (16, 36) eines biologisch verträglichen Polymers, die an ihren entgegengesetzten Enden mit einem Bindefilm (14, 34) aus einem biologisch verträglichen Bindeharz, in welchem die Enden der Stränge eingebettet sind, miteinander verbunden sind, und eine Schutzhülle (22, 42) aus biologisch verträglichem Polymermaterial, die die Stränge zwischen ihren verbundenen Enden umgibt und an wenigstens einem Ende mit dem Bindeharzfilm verbunden ist, besitzt, wobei die Stränge (16, 36) zwischen ihren verbundenen Enden gegeneinander und bezüglich der umgebenden Hülle leicht gleitbar sind.

2. Zugglied nach Anspruch 1, dadurch gekennzeichnet, daß die mehreren parallelen Stränge (16, 36) von den Kettsträngen einer Länge eines Gewebes aus biologisch verträglichen Fasern gebildet werden, dessen Enden von den Filmen (13, 34) aus Bindeharz miteinander verbunden sind und dessen Schußstränge aus der Gewebelänge zwischen den gebundenen Enden entfernt wurden, so daß sie die Kettstränge in getrennter paralleler Anordnung hinterlassen.

3. Zugglied nach Anspruch 1 oder 2, dadurch gekennzeichnet, daß die parallelen Stränge (16, 36) durch Beschichten mit einer Oberflächenbeschichtung aus biologisch verträglichem Polytetrafluoräthylenharz geschmiert sind.

4. Zugglied nach Anspruch 1, 2 oder 3, dadurch gekennzeichnet, daß die parallelen Stränge (36) zu einer Endlosschliefe (40) geformt und an ihren entgegengesetzten Enden mit einem gemeinsamen Film (34) aus biologisch verträglichem Bindeharz, in welchem die Enden der Stränge eingebettet sind, miteinander verbunden sind, wobei die Schliefe in die Hülle (42) derart eingefügt ist, daß ihre entgegengesetzten Enden daraus hervortreten und Verankerungsschliefen für die Befestigung des Zuggliedes bei der Herstellung des Implantats bilden.

5. Zugglied nach Anspruch 4, worin eines oder beide der freiliegenden Enden in Eingriff mit einem Verankerungsteil (50) aus porösem biologisch verträglichem Polymermaterial stehen, das das Einwachsen von Gewebe in das Material bei der Anordnung in situ fördert.

6. Zugglied nach einem der Ansprüche 1 bis 5, dadurch gekennzeichnet, daß die Schutzhülle (22) aus einem porösen biologisch verträglichen Polymer besteht.

7. Zugglied nach Anspruch 6, dadurch gekennzeichnet, daß die Außenoberfläche der Schutzhülle (22) so behandelt ist, daß sie dem Einwachsen von Gewebe in die Hülle widersteht.

8. Zugglied nach Anspruch 7, bei dem die Außenoberfläche der Hülle (22) erhitzt wurde.

9. Zugglied nach Anspruch 7, bei dem wenigstens die Oberflächenporen der Hülle (22) mit Silikonkautschuk verschlossen sind.

10. Zugglied nach Anspruch 8, bei dem die poröse Hülle im wesentlichen vollständig mit Silikonkautschuk imprägniert ist.

11. Zugglied nach Anspruch 6, bei dem die Schutzhülle (22) eine offene poröse Oberfläche hat, die das Einwachsen von Gewebe in die Hülle fördert.

12. Zugglied nach einem der Ansprüche 6 bis 11, bei dem die Schutzhülle (22) aus einem biologisch verträglichen Polymermaterial gebildet ist, das Polytetrafluoräthylenfasern in einer porösen Matrix von Polytetrafluoräthylenbindeharz umfaßt.

13. Zugglied nach einem der Ansprüche 1 bis 5, bei dem die Hülle aus Silikonkautschuk besteht.

14. Zugglied nach einem der vorausgehenden Ansprüche, bei dem die parallelen Stränge (16, 36) aus Polyamid, Polyimid, Polytetrafluoräthylen oder Polyester bestehen und der Bindefilm (14, 34) ein Perfluorkohlenstoffpolymer, ein Perfluoralkoxyfluorkohlenstoffpolymer oder ein Polyäthylen mit hohem Molekulargewicht ist.

15. Zugglied nach einem der Ansprüche 1 bis 3 oder 6 bis 14, bei dem die Enden der Struktur nahe den Bindefilmen (12) mit einer Schicht (20) aus porösem biologisch verträglichem Polymermaterial beschichtet sind, das das Einwachsen von Gewebe in die Poren des Polymermaterials fördert.

16. Zugglied nach Anspruch 5 oder 15, bei dem das poröse biologisch verträgliche Polymermaterial ein Gemisch von Polytetrafluoräthylen und Kohlenstoffasern in einer porösen Polytetrafluoräthylenharzmatrix umfaßt.

17. Zugglied nach Anspruch 5 oder 15, bei dem das poröse Polymermaterial ein Polyesterfaservelours ist.

18. Zugglied nach einem der vorausgehenden Ansprüche, dadurch gekennzeichnet, daß wenigstens ein Ende der Hülle (22) durch eine Schutzeinfassung (26) aus biologisch verträglichem Material geschützt ist, die um die Hülle herum befestigt ist und sich entlang wenigstens eines Teils von deren Länge erstreckt.

19. Zugglied nach Anspruch 18, dadurch gekennzeichnet, daß die Schutzeinfassung (26) ein rohrförmiges Teil umfaßt, das koaxial mit dem Ende der Hülle (22) sich erstreckt und mit ihm verbunden ist.

20. Zugglied nach einem der vorausgehenden Ansprüche, dadurch gekennzeichnet, daß wenigstens ein Ende der Struktur außen durch eine entfernbar Hülse (28) geschützt ist, um die Hülle vor oder während der Implantation gegen Verletzung zu schützen, und die von der Struktur nach der Implantation entfernbar ist.

21. Zugglied nach Anspruch 20, dadurch gekennzeichnet, daß das außen geschützte Ende der Struktur eine verminderte Abmessung besitzt, um eine Führung (24) für das Einsetzen der Struktur während der Implantation zu bilden, wobei die Hülse (28) an diese Führung gebunden ist und mit ihr nach der Implantation der Struktur entfernbar ist.

FIG.1

FIG.2

FIG.3

FIG.6

FIG.4

FIG.5